(19) Europäisches Patentamt
European Patent Office
Office européen des brevets

(11) Publication number: 0 010 119 A1

(12) **EUROPEAN PATENT APPLICATION**

(21) Application number: 79100254.6

(22) Date of filing: 29.01.79

(51) Int. Cl.³: C 07 D 217/20
C 07 C 57/34, C 07 C 57/42
A 61 K 31/47

(30) Priority: 21.07.78 US 926794

(43) Date of publication of application: 30.04.80 Bulletin 80/9

(84) Designated Contracting States: BE CH DE FR GB IT LU NL SE

(71) Applicant: THE MASSACHUSETTS GENERAL HOSPITAL
55 Fruit Street
Boston Massachusetts 02114(US)

(72) Inventor: Savarese, John Joseph
84 Balmoral Road
Boxford, Massachusetts(US)

(72) Inventor: Kitz, Richard John
6 Pond Road
Dover, Massachusetts(US)

(72) Inventor: Ginsburg, Sara
85 West End Avenue
New York N.Y.(US)

(74) Representative: Berg, Wilhelm, Dr. et al,
Dr. Berg, Dipl.-Ing. Stapf, Dipl.-Ing. Schwabe, Dr. Dr. Sandmair Patentanwälte Postfach 860245
D-8000 München 80(DE)

(54) **Isoquinoline derivatives, their preparation, pharmaceutical compositions containing these compounds and intermediates.**

(57) Intermediate-duration reversible neuromuscular blocking agents of the formula (I)

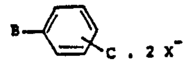

(I)

where B and C are preferably para or may be meta and are each

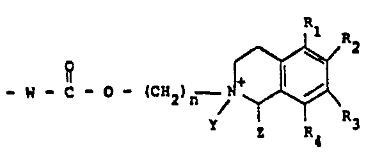

where W is $CH_2$ or most preferably CH = CH $R_1$, $R_2$, $R_3$ and $R_4$ are the same or different and are each hydrogen or lower alkoxy of 1 to 4 carbon atoms and preferably methoxy, Y is lower alkyl of 1 to 4 carbon atoms and preferably methyl, Z is hydrogen, lower alkyl of 1 to 4 carbon atoms, cyclopentyl, cyclohexyl, benzyl or

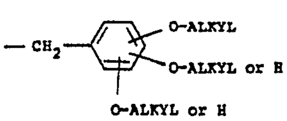

where ALKYL has 1 to 4 carbon atoms preferably where the O-ALKYL is at the 2, 3, 4 or 5 positions such as 4-methoxy benzyl and is most preferably 3, 4-dimethoxy benzyl or 3, 4, 5-trimethoxybenzyl, n is 2, 3 or 4, mos preferably 2 or 3 provided that at least one of $R_1$ to $R_4$ is lower alkoxy and most preferably where $R_1$ and $R_4$ is hydrogen and $R_2$ and $R_3$ are methoxy and X is a pharmaceutically acceptable anion.

The neuromuscular blocking agents of formula I are useful for administration to a patient to cause skeletal muscle relaxion during surgery and are normally administered intravenously in a pharmaceutically acceptable carrier.

EP 0 010 119 A1

TITLE MODIFIED
see front page

# CHEMICAL COMPOUNDS, METHODS AND PREPARATION

## Background of the Disclosure

In anesthesia, neuromuscular blocking agents are used to provide skeletal muscular relaxation during surgery and during intubation of the trachea. In general there are two types of neuromuscular blocking agents in use, nondepolarizing and depolarizing. The nondepolarizing agents include d-tubocurarine, pancuronuim gallamine, diallyltoxiferine, and toxiferine.

The depolarizing agents include succinylcholine and decamethonium. All of the conventional nondepolarizing agents when used for producing skeletal muscle relaxation in surgery have a long duration of action e.g., 60 to 180 minutes in man. The depolarizing agents on the other hand provide muscle relaxation at dosages normally used for surgery which is less than the duration of action of nondepolarizing agents.

For example, succinylcholine provides a short duration of action of about 5 to 15 minutes whereas decamethonium provides about 20 to 40 minutes duration of muscle relaxation. To the best of applicants' knowledge there are no nondepolarizing agents currently available for approved clinical use which have an intermediate duration of action. As used herein, an intermediate duration of action is defined as about 15 to 30 minutes in cats and monkeys.

The long duration of action of nondepolarizing agents is unacceptable in many surgical procedures which take less

than one hour because the patient is not generally fully recovered from their effects e.g., the patient may be unable to breathe adequately on his or her own.

Each nondepolarizing agent has inherent side-effects. For example, allamine and pancuronium may cause tachycardia, and d-tubocurarine and diallyltoxiferine may cause hypotension. While such drugs can be pharmacologically antagonized with anticholinesterase agents, this obviously necessitates the administration of a second drug which itself may have its own side effects e.g., bradycardia, gut spasm and bronchorrhea. Thus to overcome the aforementioned side-effects of the anticholinesterase agents, a third drug, an anticholinergic drug e.g., atropine must also be given.

The depolarizing agents to the best of applicants' knowledge have no pharmacological antagonists. While in most cases there is no need to reverse the effects of the depolarizing agents, in certain patients the effects are much prolonged because of abnormal metabolism of the agent by the patient.

The depolarizing agents due to that mode of action which initially causes skeletal muscle contraction and stimulation of smooth muscles are also known to cause the following side-effects in certain instances; increased intraocular, and intragastric tension, cardiac arrhythmisas, potassium release, and muscle pain. These side-effects caused by the depolarizing agents are not caused by the nondepolarizing agents. It is therefore clearly evident that a new neuromuscular blocking agent having the relatively few side-effects and the reversibility of the nondepolarizing agents yet being

of considerably shorter i.e., intermediate, duration of action is needed. No such drug is in clinical use at the present time.

It should be understood that while nondepolarizing agents generally have few side-effects, gallamine and pancuronium may cause tachycardia and d-tubocurarine and diallyltoxiferine may cause hypotension. Surprisingly, the compounds of the present invention also appear to be free of these side-effects at the dosages anticipated being used clinically in tests made to date. Reference may be had to the next of: "The Pharmacological Basis of Therapeutics" - Fifth Edition, edited by Louis S. Goodman and Alfred Gilman published by The McMillian Co., Copyright 1975, Chapter 28, author George B. Koelle, for a further description of neuromuscular blocking agents.

Reference should also be had to the following articles: "Neuromuscular Blocking Activity of a New Series of Quaternary N-Substituted Choline Esters" - British Journal of Pharmacology, September 1971, vol. 43, No. 1, p. 107.

"The Pharmacology of New Short Acting Nondepolarizing Ester Neuromuscular Blocking Agents:Clinical Implications" - published in Anesthesia and Analgesia .... Current Researches, Vol. 52, No. 6, p. 982 NOV.-DEC., 1973;

"Potential Clinical Uses of Short-Acting Nondepolarizing Neuromuscular-Blocking Agents as Predicted from Animal Experiments" - published in Anesthesia and Analgesia ... Current Researches, Vol. 54, No. 5, Sept.-Oct., 1974;

"U.S. Patent No. 3,491,099" for a further description of neuromuscular blocking agents; and

"Does Clinical Anesthesia Need New Neuromuscular Blocking Agents?" - published in Anestesiology, Vol. 42, No. 3, March 1975, P. 236.

## Brief Description of the Disclosure

The present invention provides new and improved neuromuscular blocking agents sometimes called muscle relaxants which combine a nondepolarizing mode of action with the intermediate duration of action and reversibility reeded to meet improved clinical reqzirements for use during surgery.

The intermediate-duration reversible neuromuscular blocking agents of the formula (I)

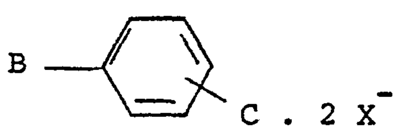

(I)

where B and C are preferably para or may be meta and are each

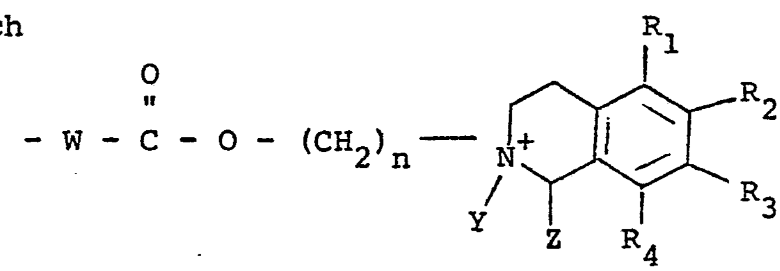

where W is $CH_2$ or CH = CH
$R_1$, $R_2$, $R_3$ and $R_4$ are the same or different and are each hydrogen or lower alkoxy of 1 to 4 carbon atoms and preferably methoxy, Y is lower alkyl of 1 to 4 carbon atoms and preferably methyl, Z is hydrogen, lower alkyl of 1 to 4 carbon atoms, cyclopentyl, cyclo-hexyl, benzyl, or

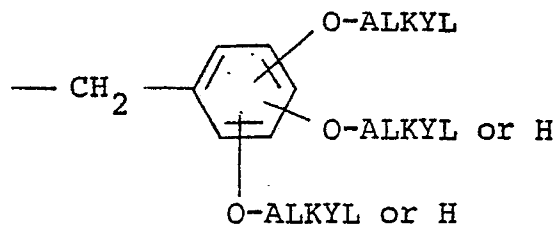

where ALKYL has 1 to 4 carbon atoms preferably where the O ALKYL is at the 2, 3, 4 or 5 positions such as 4-methoxy benzyl and is most preferably 3, 4-dimethoxy benzyl or 3, 4, 5-trimethoxybenzyl, n is 2, 3 or 4, most preferably 2 or 3 provided that at least one of $R_1$ to $R_4$ is lower alkoxy and most preferably where $R_1$ and $R_4$ is hydrogen and $R_2$ and $R_3$ are methoxy and X is a pharmaceutically acceptable anion.

In the above alkyl of 1 to 4 carbon atoms is meant to include branched or straight chain alkyl (e.g., methyl, ethyl, propyl, butyl, etc.,) and alkoxy of 1 to 4 carbon atoms is meant to include methoxy, ethoxy, propoxy and butoxy. Of the compounds of the invention the most preferred are the compounds of the formula II

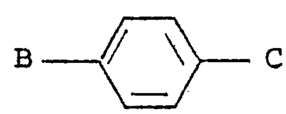

(II)

where B and C are as defined above, where
W is $CH_2$ or CH = CH
n is 3, Y is methyl and Z is 3, 4-dimethoxy benzyl or 3, 4, 5-trimethoxy benzyl, $R_1$ and $R_4$ are hydrogen and $R_2$ and $R_3$ are methoxy.

Of the compounds of special note there is mentioned the following with the substitutions as set forth below based on the structure of formula II and identified as follows:

(KK-100) n is 3, Y is methyl, W is $CH_2$, $R_1$ and $R_4$ are hydrogen, $R_2$ and $R_3$ are methoxy and Z is 3, 4-dimethoxybenzyl;

(LL46) n is 3, Y is methyl, W is $CH_2$, $R_1$ and $R_4$ are hydrogen, $R_2$ and $R_3$ are methoxy and Z is 3, 4, 5-trimethoxybenzyl;

(HH109) n is 3, Y is methyl, W is CH = CH, $R_1$ and $R_4$ are hydrogen, $R_2$ and $R_3$ are methoxy and Z is 3, 4, 5-trimethoxybenzyl where B is para to C and (LL39) where the substituents are the same as in (HH109) and B is meta to C.

(GG195) is 3, Y is methyl, W is CH = CH, $R_1$ and $R_4$ are hydrogen, $R_2$ and $R_3$ are methoxy and Z is 3, 4-dimethoxybenzyl.

The above specifically mentioned compounds are most preferred as intermediate duration compounds in that they have relatively low but still measurable hydrolysis rates which distinguishes them from short acting neuromuscular blocking agents. The compounds where W is CH = CH are most preferred because of both their activity and few side effects and most particularily the compounds (HH109) and (LL39), are by far the best at this time since they exhibit fewest side-effects and very high potency.

Of the anions of the invention, the following are examples of those which are suitable: iodide, mesylate,

tosylate, bromide, benzene sulfonate, nitrobenzene sulfonate, naphthylene sulfonate, chloride, sulfate, phosphate, hydrogen phosphate acetate and propionate. The mesylate and chloride cations are most preferred because of the solubility of the salt made therefrom in water. Since the activity is in the cation portion of the compound, the nature of the anion is inimportant as long as it is pharmaceutically acceptable.

The compounds of formula I or II are used as neuromuscular blocking agents in conjunction with surgery of for intubation of the trachea by conventional parenteral administration e.g., intramuscular or intravenous administration in solution. The compounds of the present invention shown in formular I or II are administered to patients such as monkeys and man (humans) and other mammals to achieve a neuromuscular block. The dosage for each type of patient will vary because of the peculiarities of the species, however, a suitable intravenous amount or dosage of the compounds of formula I or II for a monkey would be 0,05 to 0,8 mg/kg of body weight, and for a man 0,05 to 0,8 mg/kg of body weight, and most preferably 0,1 to 0,5 mg/kg of body weight, the above being based on the weight of the action which is the active ingredient.

The compounds of this invention would normally be readministered every 15 to 30 minutes after initial administration or given as a slow continuous infusion depending upon the length of time a muscular block is desired, and as determined by the anesthetist and surgeon in charge of the patient. The compounds of this invention are reversible using conventional anticholinesterase agents such as neostigmine and edrophonium and appear to avoid the side-effects associated with the depolarizing agents.

The compounds of formula I or II are therefore useful for producing an intermediate duration neuromuscular blockage, and the present invention provides a method of producing such blockade in mammals e.g., man or monkeys, by intravenously injecting a dose of 0,05 to 0,8 mg/kg to the mammal.

The compounds may be presented in a pharmaceutical formulation for parenteral administration. The formulation may be an aqueous on non-aqueous solution or emulsion in a pharmaceutically acceptable liquid or mixture of liquids, which may contain bacteriostatic agents, antioxidants, buffers, thickening agents, suspending agents or other pharmaceutically acceptable additives.

Such formulations are normally presented in unit dosage forms such as ampoules or disposable injection devices, or in multidose forms such as a bottle from which the appropriate dose may be withdrawn. All such formulations should be rendered sterile.

The compounds of this invention may be presented as a powder e.g., as a unit dose in a sealed vial to which sterile water may be added by a needle. A suitable unit dose to obtain a neuromuscular block for mammals e.g., humans or monkeys is about 1 mg ot 100 mg and most preferably 3 to 50 mg.

Thus a suitable pharmaceutical parenteral preparation will preferably contain 20 to 100 mg of the compounds of formulas I or II of this invention in solution. A pharmaceutical formulation may conveniently contain 5 to 400 mg, preferably 10 to 400 mg, and most preferably 5

to 200 mg of the compounds of this invention. A simple and preferred formulation is a solution of the compound of formula I or II in water which may be prepared by simply dissolving the compound into previously sterilized pure, i.e., hydrogen free water under aseptic conditions and sterilizing the solution.

The compound of formula I or II may also be administered as an infusion of a dextrose solution or a saline solution e.g., Ringers' Solution. The compounds (formulas I or II) of this invention may be prepared by the following methods:

Method 1

Appropriately substituted tetrahydroisoquinolines are prepared in the customary fashion from appropriately substituted phenylethylamines and phenylactic acids by the Bischler-Napieralski reaction. The tertiary tetrahydroisoquinoline is quaternized with an appropriate $\alpha$-bromo $\omega$-chloro, $\alpha$-iodo $\omega$-chloro, or $\alpha$-iodo $\omega$-bromo alkane. The resulting N-methyl, N-($\omega$-haloalkyl) tetrahydroisoquinolinium halide is boiled in water with the silver salt of the appropriate dicarboxylic acid, yielding silver bromide and the benzylisoquinolinium salt of the acid. This salt rearranges to the corresponding ester on heating: for example, the general reaction using $\alpha$-bromo $\omega$-chloro alkane is illustrated as follows:

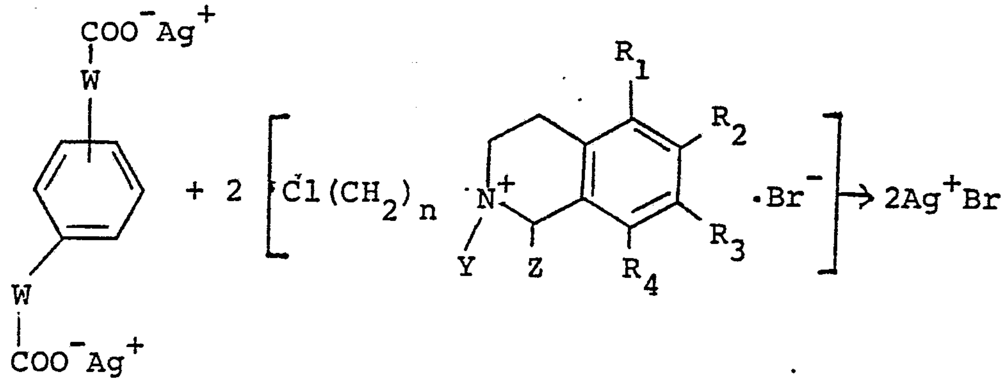

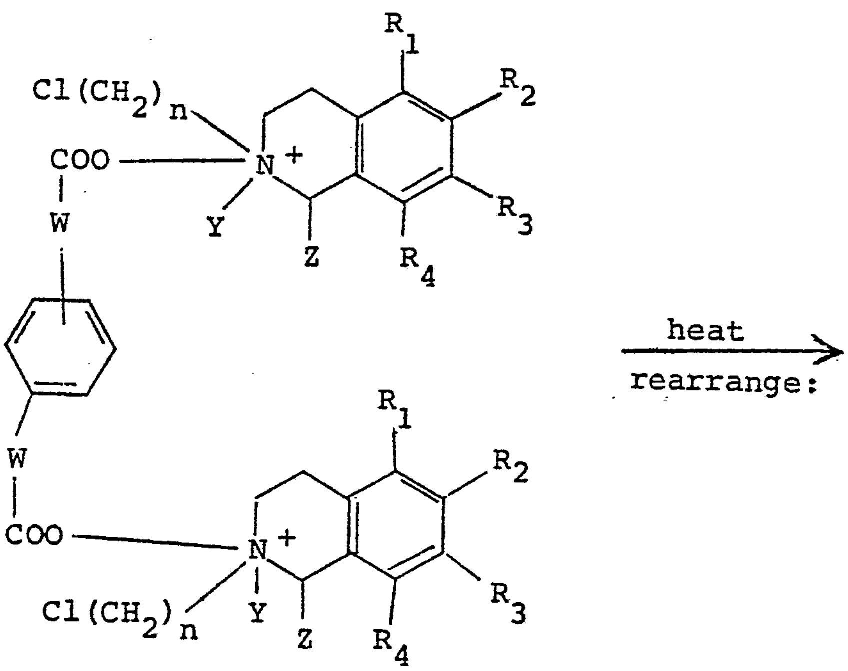
heat
rearrange:

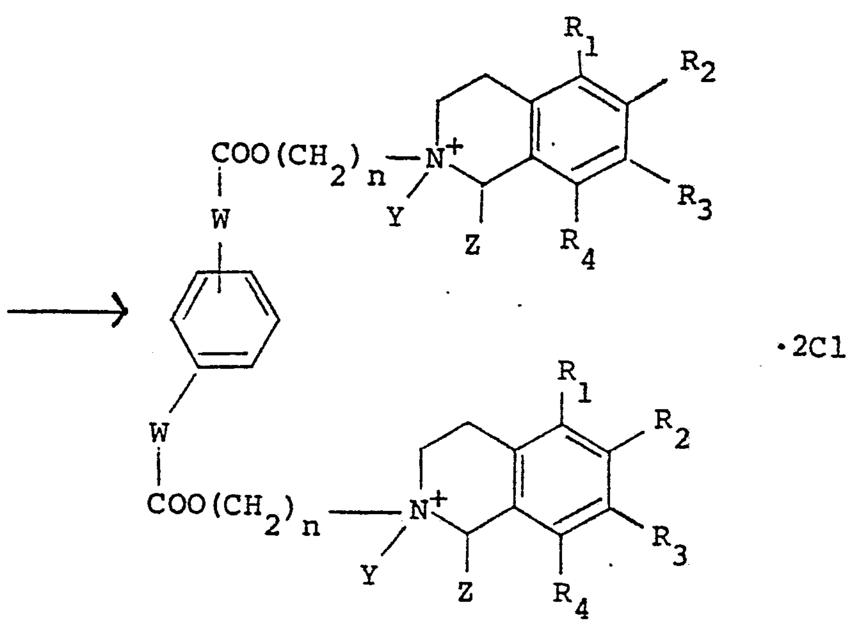

where W = $CH_2$ or CH = CH, and n, Y, Z and $R_1$ to $R_4$ are as previously defined. Other salts are prepared by conventionally reacting the dichloro salt in an ion exchange reaction with an appropriate salt of the desired anion e.g., silver mesylate, silver tosylate, etc.

Method 2

The bis-acid chloride of an appropriate phenylene dicarboxylic acid is prepared in the usual fashion by treatment of the acid with thionyl chloride. The acid chloride is esterified with an appropriate α-hydroxy-ω-iodoalkane, yielding the desired phenylene diacyl bis-ω-iodoalkyl ester:

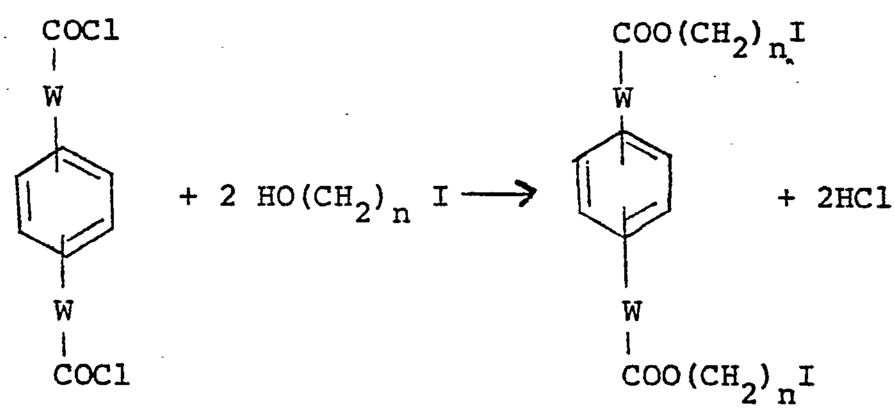


The diiodoester is refluxed with an excess of e.g., two moles of an appropriate tetrahydroisoquinoline prepared in standard fashion by the Bischler-Napieralski reaction as described in Method I. The desired bis-tetrahydroisoquinolinium diiodide (disalt) is obtained.

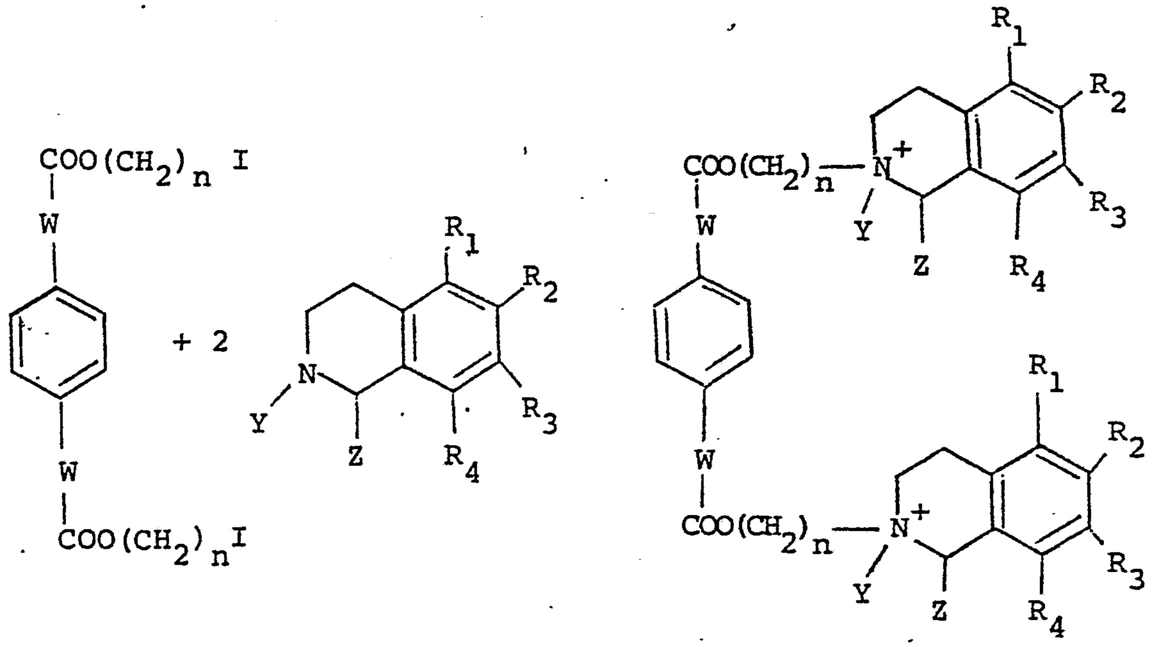

where W is $CH_2$ or CH = CH and n, Y, Z, and $R_1$ to $R_4$ are defined as above. The desired salts are then prepared in a conventional ion exchange reaction as described in Method I.

The following examples illustrate the invention. Temperatures are in degrees centigrade.

EXAMPLE 1

Preparation of Bis-3-[N-methyl-1-(3,4,5-trimethoxybenzyl)] 6,7-dimethoxy-1,2,3,4-tetrahydroisoquinolinium propyl p-phenylene-3,3'-diacrylate dichloride (HH109)

1. Preparation of silver p-phenylene diacrylate

| | |
|---|---|
| p-phenylene diacrylate acid 4,4 qm = | 40 meq |
| $H_2O$ | 60 ml |
| KOH 1N | 40 ml |

The mixture is heated to boiling, and, if necessary, the pH is adjusted to 7,0 with the same acid. $AgNO_3$ 6,8 gm = 40 m M is added to the yellow hot solution. Immediately a heavy precipitate forms. The mixture is cooled and filtered and the filter cake is washed with water, refiltered and dried. Yield = quantitative. The product is an amorphous, slightly colored powder. It is pulverised for use in the next step.

2. Preparation of 5'-Methoxylaudanosine

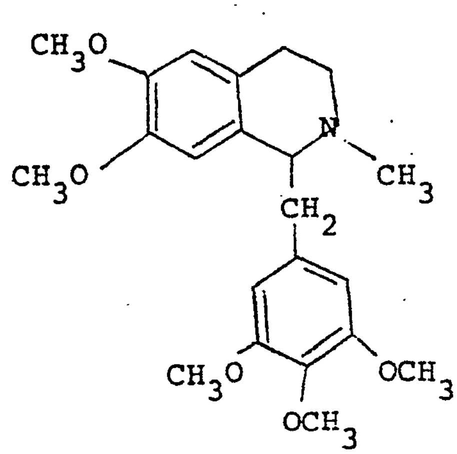

3,4-dimethoxyphenylethylamine and 3,4,5-trimethoxyphenylacetic acid are heated together at 165 - 190° in a flask until bubbling of water subsides. The product, 3,4,5-trimethoxybenzylacetylhomoveratrylamine, is recrystallized from methanol. Yield = 80 %. m.p. = 94°.

1,9 gm (10 mM) 3,4,5-trimethoxybenzylacetylhomoveratrylamine is refluxed in 15 ml toluene together with 5 ml $POCl_3$ for 2 hours. The settled semisolids are carefully separated ($POCl_3$ excess!) and the free base liberated by adding excess of NaOH and extracted with benzene. The product, 6,7-dimethoxy-1-(3',4',5'-trimethoxybenzyl) 3,4-dihydroisoquinoline is refluxed in acetone or benzene with an excess of methyl iodide. The quaternary salt, 6,7-dimethyl-1-(3',4',5'-trimethoxybenzyl)2-methyl 3,4-dihydroisoquinolinium iodide, precipitates out. m.p. = 224°.

1 gm (10 mM) 6,7-dimethoxy-1-(3',4',5'-trimethoxybenzyl)2-methyl 3,4-dihydroisoquinolinium iodide is dissolved in 80 ml $H_2O$ and 16 ml concentrated HCl. Zinc dust (1,1 gm) is added in small portions to the boiling stirred solution. The yellow color disappears (reaction time 15 - 20 minutes). The mixture is filtered hot from some unreacted zinc and rendered alkaline with concentrate NaOH. It is impractical to filter the partly precipitated zinc hydroxide, so to avoid emulsions, the whole mixture is carefully shaken with chloroform. The residue of the chloroform solution is redissolved in ether and the ether insolubles are filtered off. The ether residue does not crystallize on standing. This amine is a gummy material which hardens on standing. The crude amine is used the next step.

3. Preparation of N-(3-chloropropyl)5'-methoxylaudanosinium bromide

5'-Methoxylaudanosine 1,4 gm = 4 mM is dissolved in 8 ml dimethylformamide by warming slightly. 1-bromo-3-chloropropane 1,2 gm (about 100 % excess) is added and the mixture is left at room temperature for 5 days. (Sometimes part of the unreacted 5'-methoxylaudanosine crystallizes out, but eventually it redissolves).

The reddish-orange solution is treated with a large amount of ether and the precipitated gummy quaternary salt is decanted and slurried in fresh ether. After standing in ether for the day, low melting solids are obtained. Yield = 1,6 gm, about 80 % of theory.

4. Preparation of p-phenylene diacrylic diester of N-propyl-5'-methoxylaudanosine (HH109) (Horenstein - Pahlicke Ester Formation)

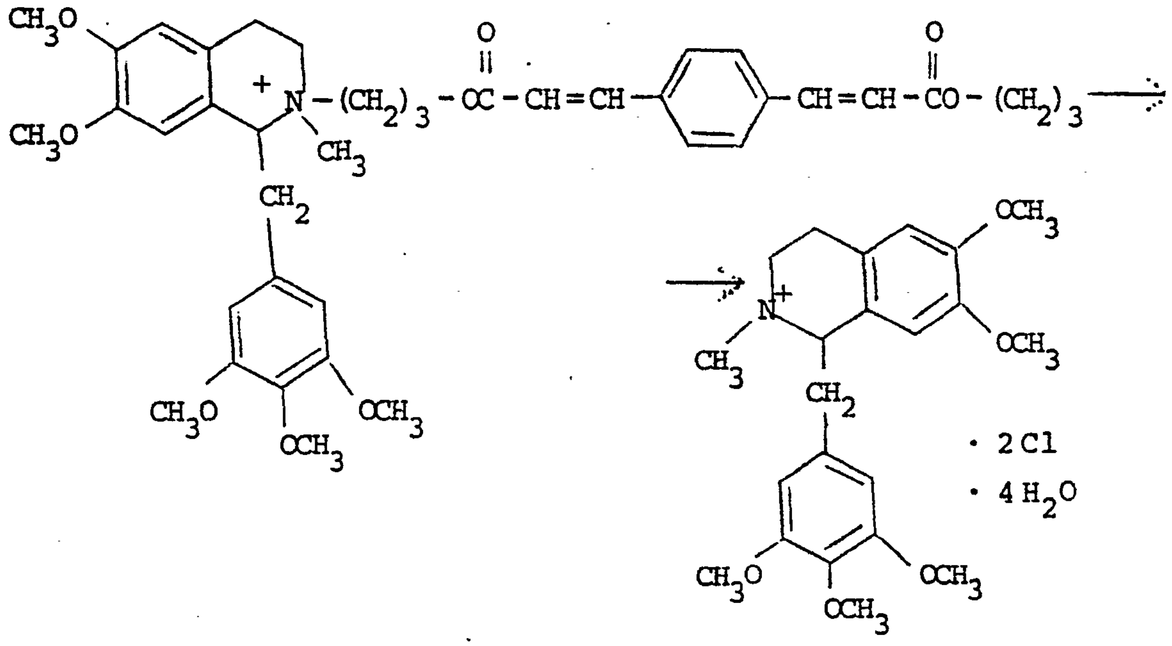

HH-109

N-(3-chloropropyl)5'-methoxylaudanosinium bromide 2,1 gm = 4 mM

| | | |
|---|---|---|
| Silver p-phenylene diacrylate | 0,85 gm = 4 mM |
| $H_2O$ | about 150 ml |

The mixture is boiled in an open beaker for about 10 - 15 minutes, stirring by hand from time to time. At the boiling temperature the silver salt is slightly soluble and reacts with the quaternary bromide. The mixture is cooled to room temperature, filtered straight and the aqueous solution is evaporated to dryness in a large dish on a steam bath. Continued heating of the residue is done for about 2 hours, after which rearrangement to the ester is complete.

The amorphous residue is boiled with isopropranolol (about 40 ml) and filtered hot from some trace mechanical impurities. Gums precipitate from the filtrate at room temperature and the precipitation is completed at about $-3^{o}$ overnight. The supernatant is decanted and the material is slurried in ethyl acetate twice.

By now the gum is semisolid and can be filtered off. After careful drying at $75^{o}$ the gums become solids. At this stage they still probably retain water in varying degrees. Yield = 1,0 gm (about 40 %). Yields vary from batch to batch. M.P. = 90 - $110^{o}$ (decomposes).

EXAMPLE 2

Preparation of Bis-3-(N-methyl-1-(3,4,5-trimethoxybenzyl) 6,7-dimethoxy-1,2,3,4-tetrahydroisoquinolinium) propyl p-phenylene-3,3'-diacetate dichloride (LL46)

1. Preparation of silver p-phenylene diacetate

| | |
|---|---|
| p-phenylene diacetic acid 4,4 gm = 40 meq purchased from Aldrich | |
| $H_2O$ | 60 ml |
| KOH 1N | 40 ml |

The mixture is heated to boiling, and, if necessary, the pH is adjusted to 7.0 with the same acid. $AgNO_3$ 6.8 gm = 40 mM is added to the yellow hot solution. Immediately a heavy precipitate forms. The mixture is cooled and filtered and the filter cake is washed with water, refiltered and dried. Yield = quantitative. The product is an amorphous, slightly colored powder. It is pulverized for use in the next step.

2. Preparation of 5'-methoxylaudanosine

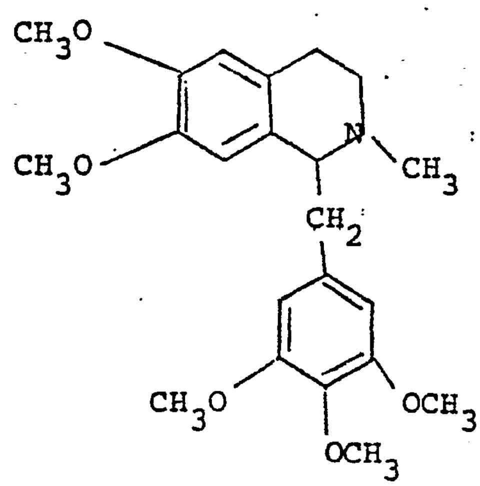

3,4-dimethoxyphenylethylamine and 3,4,5-trimethoxyphenylacetic acid are heated together at 165 - 190° in a flask until bubbling of water subsides. The product, 3,4,5-trimethoxybenzylacetylhomoveratrylamine, is recrystallized from methanol. Yield = 50 %. m.p. = 94°.

3,9 gm (10 mM) 3,4,5-trimethoxybenzylacetylhomoveratrylamine is refluxed in 15 ml toluene together with 5 ml $POCl_3$ for 2 hours. The settled semisolids are carefully separated ($POCl_3$ excess!) and the free base liberated by adding excess of NaOH and extracted with benzene. The product, 6,7-dimethoxy-1-(3',4',5'-trimethoxybenzyl)3,4-dihydroisoquinoline is refluxed in acetone or benzene with an excess of methyl iodide. The quaternary salt, 6,7-dimethoxy-1-(3',4',5'-trimethoxybenzyl)2-methyl 3,4-dihydroisoquinolinium iodide, precipitates out. m.p. = 224°.

1 gm (10 mM) 6,7-dimethoxy-1-(3',4',5'-trimethoxybenzyl)-2-nethyl 3,4-dihydroisoquinolinium iodide is dissolved in 50 ml $H_2O$ and 16 ml concentrated HCl. Zinc dust (1,1 gm) is added in small portions to the boiling stirred solution. The yellow color disappears (reaction time 15 - 20 minutes). The mixture is filtered hot from some unreacted zinc and rendered alkaline with concentrated NaOH. It is impractical to filter the partly precipitated zinc hydroxide, so to avoid emulsions, the whole mixture is carefully shaken with chloroform. The residue of the chloroform solution is redissolved in ether and the ether insolubles are filtered off. The ether residue does not crystallize on standing. This amine is a gummy material which hardens on standing. The crude amine is used in the next step.

3. Preparation of N-(3-chloropropyl)5'-methoxylaudanosinium bromide

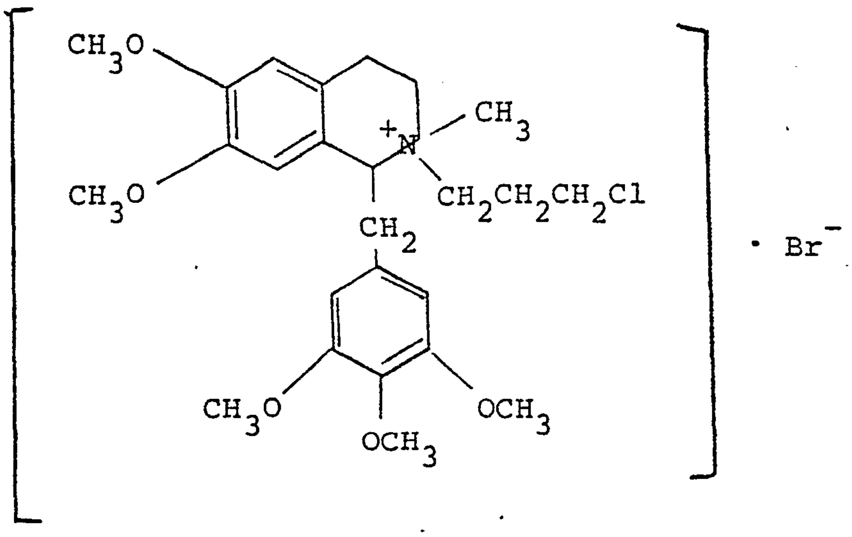

5'-Methoxylaudanosine 1,4 gm = 4 mM is dissolved in 8 ml dimethylformamide by warming slightly. 1-brome-3-choropropane 1,2 gm (about 100 % excess) is added and the mixture is left at room temperature for 5 days. (Sometimes part of the unreacted laudanosine crystallizes out, but eventually it redissolves).

The reddish-orange solution is treated with a large amount of ether and the precipitated gummy quaternary salt is decanted and slurried in fresh ether. After standing in ether for one day, low melting solids are obtained. Yield = 1,6 gm, about 80 % of theory.

0010119

4. Preparation of p-phenylene diacetic - diester of N-propyl 5'methoxylaudanosine (LL46) (Horenstein - Pahlicke Ester Formation)

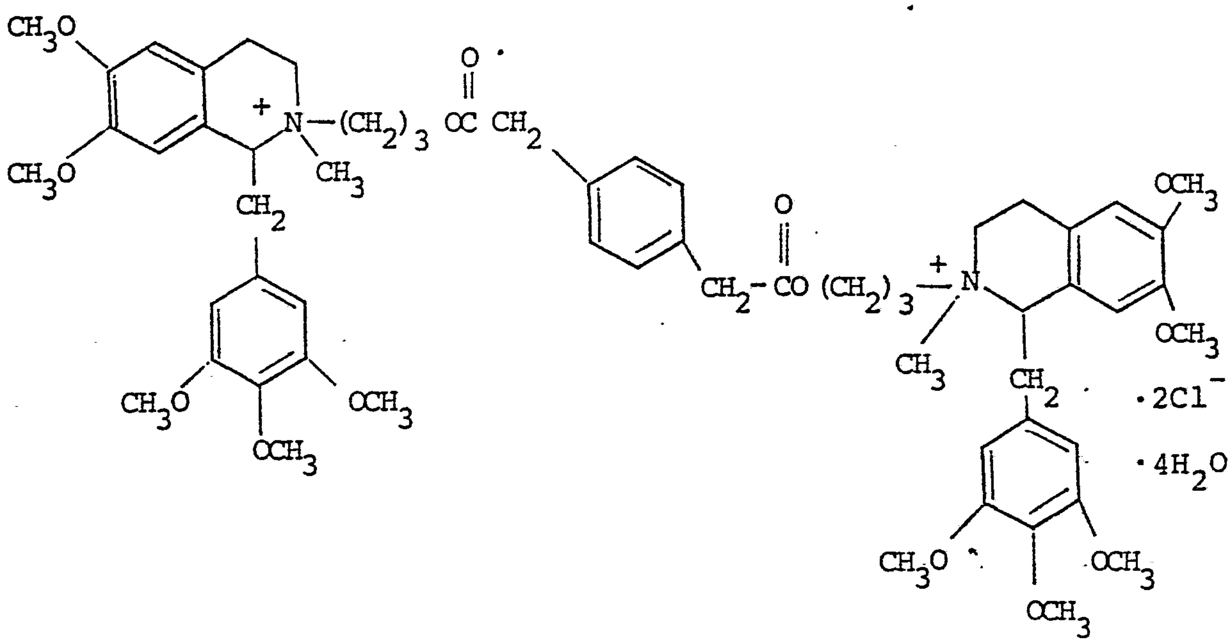

| | | |
|---|---|---|
| N-(3-chloropropyl)5'-methoxylaudanosinum bromide | | 2,1 gm=4mM |
| | Silver p-phenylene diacetate | 0,85 gm=4mM |
| | $H_2O$ | about 150 ml |

The mixture is boiled in an open beaker for about 10 - 15 minutes, stirring by hand from time to time. At the boiling temperature the silver salt is slightly soluble and reacts with the quaternary bromide. The mixture is cooled to room temperature, filtered straight and the qqueous solution is evaporated to aryness in a large dish on a steam bath. Continued heating of the residue is done for about 2 hours, after which the rearrangement to the ester is complete.

The amorphous residue is boiled with isoproprandiol (about 10 ml) and filtered hot from some trace mechanical impurities gums precipitate from the filtrate at room temperature and the precipitation is completed at about $-3^{O}$ overnight. The supernatant is decanted and the material is slurried in ethyl acetate twice. By now the gum is somisolid and can be filtered off. After careful drying at $75^{O}$ the gums become solids. At this stage they still probably retain water in varying degrees. Yield = 1,0 gm (about 40 %). Yields vary from batch to batch. M.P. = 80 - 90 % (decomposes)

EXAMPLE 3

Preparation of Bis-3-[N-methyl-1-(3,4-dimethoxybenzyl) 6,7-dimethoxy-1,2,3,4-tetrahydroisoquinolinium] propyl p-phenylene-3,3'-diacrylate dichlorid. (GG195)

1. Preparation of silver p-phenylene diacrylate

| | |
|---|---|
| p-phenylene diacrylic acid 4,4 gm = | 40 meq |
| purchased from Aldrich | |
| $H_2O$ | 60 ml |
| KOH 1N | 40 ml |

The mixture is heated to boiling, and if necessary, the pH is adjusted to 7,0 with the same acid. $AgNO_3$ 6,8 gm = 40 mM is added to the yellow hot solution. Immediately a heavy precipitate forms. The mixture is cooled and filtered and the filter cake is washed with water, refiltered and dried. Yield = quantitative. The product is an amorphous, slightly colored powder. It is pulverized for use in the next step.

2. Preparation of 3-chloropropyl laudanosinium bromide:

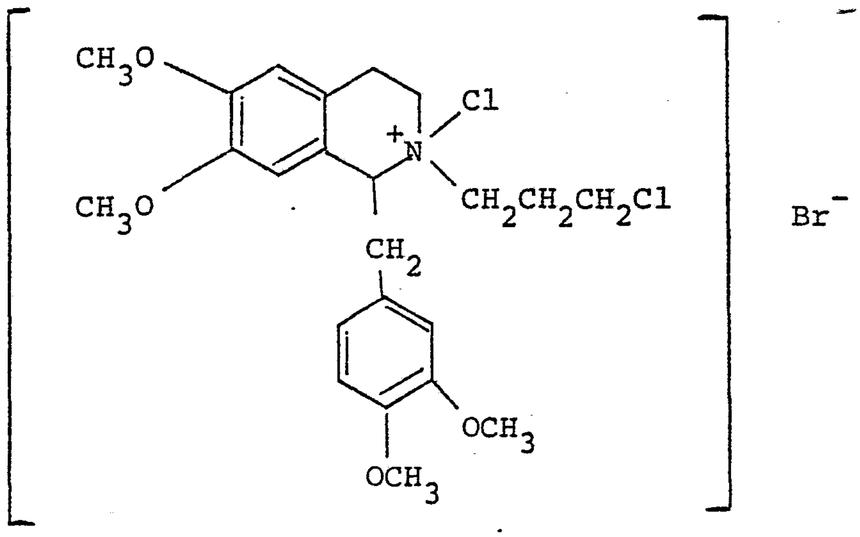

Laudanosine (Aldrich) 1,4 gm = 4 mM is dissolved in 8 ml dimethylformamide by warming slightly. 1-bromo-3-chloropropane 1,2 gm (about 100 % excess) is added and the mixture is left at room temperature for 5 days. (Sometimes part of the unreacted laudanosine crystallizes out, but eventually it redissolves).

The reddish-orange solution is treated with a large amount of ether and the precipitated gummy quaternary salt is decanted and slurried in fresh ether. After standing in ether for one day, low melting solids are obtained. Yield = 1,6 gm, about 80 % of theory.

3. Preparation of p-phenylene diacrylic diester of N-propyl laudanosine (GG195)
(Horenstein - Pahlicke Ester Formation)

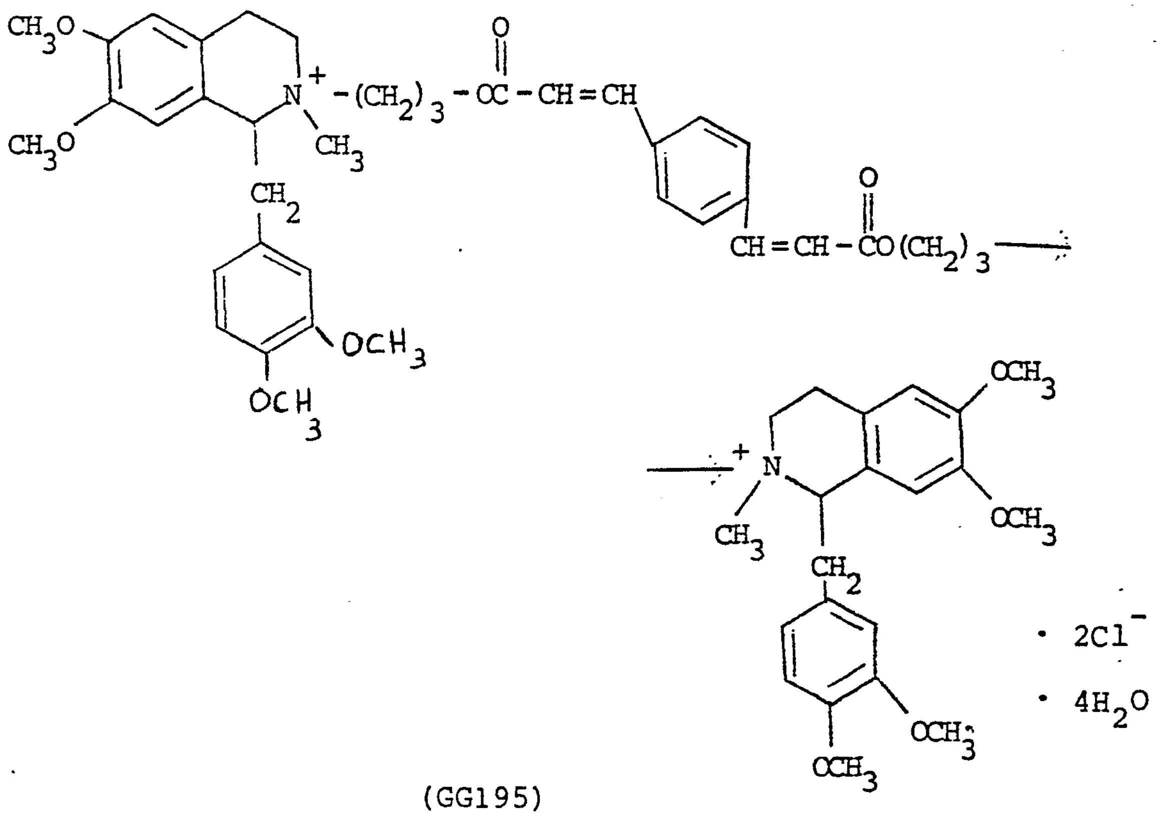

(GG195)

N-(3-chloropropyl) laudanosinium bromide 2,1 gm = 4 mM
Silver p-phenylene diacrylate 0,85 gm = 4 mM
$H_2O$ about 150 ml

The mixture is boiled in an open beaker for about 10 - 15 minutes, stirring by hand from time to time. At the boiling temperature the silver salt is slightly soluble and reacts with the quaternary bromide. The mixture is cooled to room temperature, filtered straight and the aqueous solution is evaporated to dryness in a large dish on a steam bath. Continued heating of the residue is done for about 2 hours, after which the rearrangement to the ester is complete.

The amorphous residue is boiled to isopropranol (about 40 ml) and filtered hot from some trace mechanical impurities. Gums precipitate from the filtrate at room temperature and the precipitation is completed at about $-3^{o}$ overnight. The supernatant is decanted and the material is slurried in ethyl acetate twice. By now the gum is semisolid and can be filtered off. After careful drying at $75^{o}$ the gums become solids. At this stage they still probably retain water in varying degrees. Yield = 1,0 gm (about 40 %). Yields vary from batch to batch. M.P. = 90 - $110^{o}$ (decomposes).

EXAMPLE 4

Preparation of Bis-3-[N-methyl-1-(3,4-dimethoxybenzyl) 6,7-dimethoxy-1,2,3,4-tetrahydroisoquinolinium] propy p-phenylene-3,3'-diacetate dichlorid. (KK100)

1. Preparation of silver-p-phenylene diacetate

| | |
|---|---|
| p-phenylene diacetic acid 4,4 gm = 40 meq purchased from Aldrich | |
| $H_2O$ | 60 ml |
| KOH 1N | 40 ml |

The mixture is heated to boiling, and, if necessary, the pH is adjusted to 7,0 with the same acid. $AgNO_3$ 6,8 gm = 40 mM is added to the yellow hot solution. Immediately a heavy precipitate forms. The mixture is cooled and filtered and the filter cake is washed with water, refiltered and dried. Yield = quantitative. The product is an amorphous, slightly colored powder. It is pulverized for use in the next step.

2. Preparation of 3-chloropropyl laudanosinium bromide:

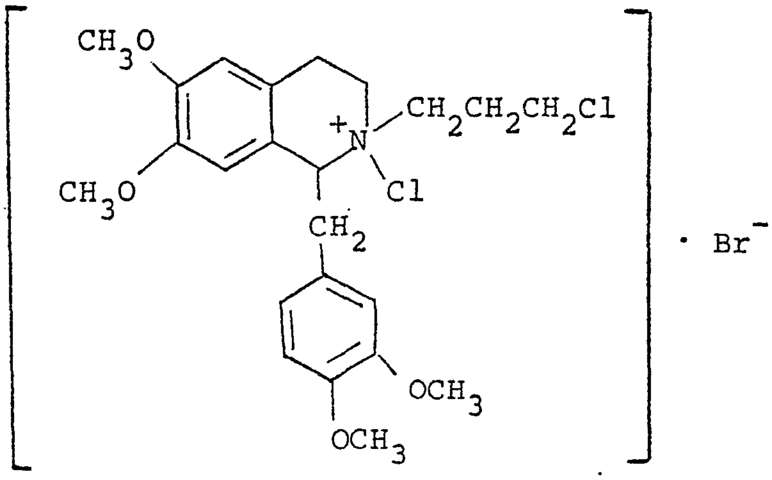

Laudanosine (Aldrich) 1,4 gm = 4 mM is dissolved in 8 ml dimethylformamide by warming slightly. 1-bromo-3-chloropropane 1,2 gm (about 100 % excess) is added and the mixture is left at room temperature for 5 days. (Sometimes part of the unreacted laudanosine crystallizes out, but eventually it redissolves).

The reddish-orange solution is treated with a large amount of ether and the precipitated gummy quaternary salt is decanted and slurried in fresh ether. After standing in ether for one day, low melting solids are obtained. Yield = 1,6 gm, about 80 % of theory.

3. Preparation of p-phenylene diacetic diester of N-propyl laudanosine (KK100) (Horenstein - Pahlicke Ester Formation)

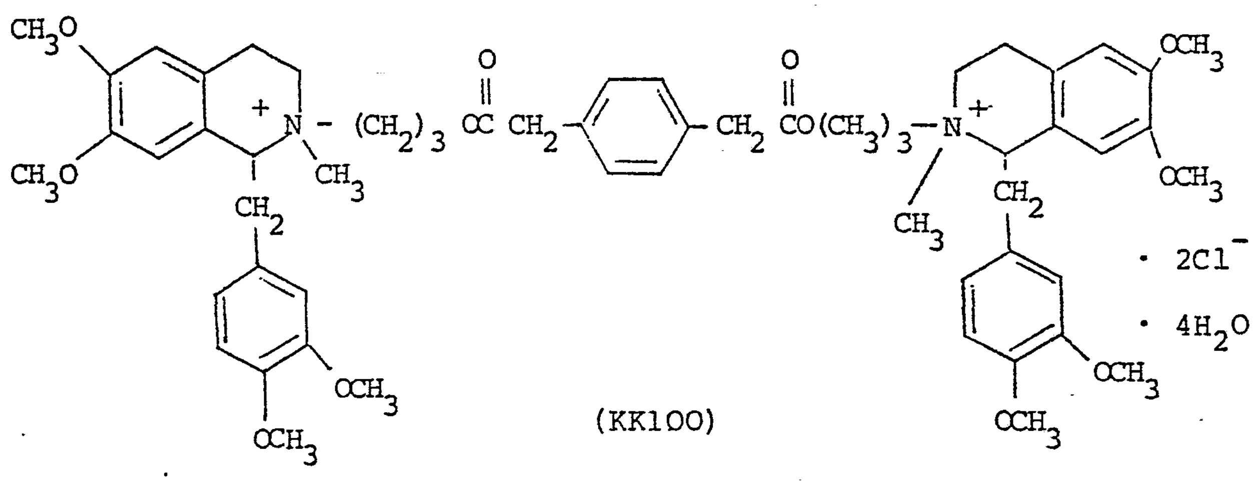

(KK100)

| | | |
|---|---|---|
| N-(3-chloropropyl) laudanosinium bromide | | 2,1 gm = 4 mM |
| Silver p-phenylene diacetate | | 0,85 gm = 4 mM |
| $H_2O$ | about | 150 ml |

The mixture is boiled in an open beaker for about 10 - 15 minutes, stirring by hand from time to time. At the boiling temperature the silver salt is slightly soluble and reacts with the quaternary bromide. The mixture is cooled to room temperature, filtered straight and the aqueous solution is evaporated to dryness in a large dish on a steam bath. Continued heating of the residue is done for about 2 hours, after which the rearrangement to the ester is complete.

The amorphous residue is boiled with isopropanol (about 40 ml) and filtered hot from some trace mechanical impurities. Gums precipitate from the filtrate at room temperature and the precipitation is completed at about $-3^{o}$ overnight. The supernatant is decanted and the

material is slurried in ethyl acetate twice. By now the gum is somisolid and can be filtered off. After careful drying at 75° the gums become solids. At this stage they still probably retain water in varying degrees. Yield = 1,0 gm (about 40 %) Yields vary from batch to batch. M.P. = 80 - 90° (decomposes).

EXAMPLE 5

Pharmaceutical formulation (HH109) us dissolved in water for injection to a concentration of 5 mg/ml. The solution is then poured into 10 ml vials which are then sealed.

EXAMPLE 6

Sterile (HH109) powder(50 mg) is aseptically packaged in 10 ml vials sealed with a rubber-stopper. Ten ml sterile water for injection is added to the vials in order to produce a cent (5 mg/ml) solution of (HH109).

EXAMPLE 7

The compounds HH1o9, GG195, KK100, LL46 were each separately dissolved 0,9 per cent saline at a concentration of 2 mg/ml. Cynomolgus monkeys are anesthetized with halothane, nitrous oxide and oxygen. The maintenance concentration of halothane was 1,0 %. Arterial and venous catheters were placed in the femoral vessels for drug administration and recording of the arterial pressure. Controlled ventilation was

accomplished via an endotrachael tube. Twitch and tetanic contractions of the tibialis arterior muscle were elicited indirectly via the sciatic nerve. Recordings of arterial pressure electrocardiogram (lead I), heart rate, and muscle function were made simultaneously.

As shown in Table 1, four to six animals received each listed compound. Four additional animals received succinylcholine chloride or d-tubocurarine chloride as controls. The chart shows the dose range required to produce 95 per cent block of the twitch response of the tibialis anterior muscle under above anestetic conditions in each series of animals receiving each drug. Also listed in the chart is the range of the duration of action of each compound in each series of animals. Duration of action is defined as the time span from drug injection to full recovery of the twitch response of the tibialis anterior muscle.

The duration of action of these compounds in monkeys is more indicative of the possible duration of action of the compounds in man than studies done in other species, such as the cat and dog, for the following reason: the compounds are believed to be broken down (hydrolyzed) by an enyzeme (plasma cholinesterase) present in man, monk4y, cat and dog. The rate of breakdown of any compound by this enzyme is believed to be the principal determinant of its duration of action in the body. The plasma cholinesterase activity of the rhesus monkey is known to be most similar to that of man (e.g., Hobbiger and Peck, British Journal of Pharmacology 37: 258 - 271, 1969).

TABLE 1

NEUROMUSCULAR BLOCKING POTENCY OF SELECTED COMPOUNDS IN THE RHESUS MONKEY

| Compound | Number of aninals tested | $ED_{95}$ * (MG/NG cation) | Range of dupation of action (minutes from injection to full recovery) |
|---|---|---|---|
| HH109 | 4 | 0,1 - 0,4 | 20 - 30 |
| GG195 | 4 | 0,2 - 0,6 | 15 - 30 |
| KK100 | 4 | 1,0 - 3,0 | 10 - 20 |
| LL46 | 4 | 0,6 - 2,0 | 15 - 25 |
| Succinyl-choline | 4 | 1,0 - 2,0 | 4 - 6 |
| l-Tube-curarine | 4 | 0,2 - 0,4 | 30 - 50 |

* $ED_{95}$ means the dose necessary to produce 95 per cent block of the twitch response of the tibialis anterior muscle stimulated indirectly at 0,15 Hz via the sciatic nerve.

EXAMPLE 8

Bis-3-[N-methyl-1-(3,4,5-trimethoxybenzyl)6,7-dimethoxy-1,2,3,4-tetrahydroisoquinolinium] propyl p-phenylene-3,3'-diacrylate dimesylate is prepared in an ion exchange reaction by reacting HH109 with silver mesylate. The dichloride HH109 is dissolved in water as is the silver mesylate. The reaction mixture is stirred to form the silver chloride precipitate. The mixture is filtered through filter paper to remove the silver chloride thereby leaving the mesylate salt in solution. The water is then evaporated.

EXAMPLE 9

Bis-3-[N-methyl-1-(3,4,5-trimethoxybenzyl)-6,7-dimethoxy-1,2,3,4-tetrahydroisoquinolinium] propyl p-phenylene-3,3'-diacrylate ditosylate is prepared in an ion exchange reaction by reacting HH109 with silver tosylate. The dichloride HH109 is dissolved in water as is the silver tosylate. The reaction mixture is stirred to form the silver chloride precipitate. The mixture is filtered through filter paper to remove the silver chloride thereby leaving the tosylate salt in solution. The water is then evaporated.

EXAMPLE 10

Preparation of Bis-3[N-methyl-1-(3,4,5-trimethoxybenzyl) 6,7, dimethoxy-1,2,3,4-tetrahydroisoquinolinium]propyl m-phenylene-3,3'-diacrylate dichlorid (LL39)

1. Preparation of silver m-phenylene diacrylate

| | | |
|---|---|---|
| m-phenylene diacrylic acid | 4,4 gm = | 40 meq |
| $H_2O$ | | 60 ml |
| KOH 1N | | 40 ml |

The mixture is heated to boiling, and, if necessary, the pH is adjusted to 7,0 with the same acid. $AgNO_3$ 6,8 gm = 40 mM is added to the yellow hot solution. Immediately a heavy precipitate forms. The mixture is cooled and filtered and the filter cake is washed with water, refiltered and dried. Yield = quantitative. The product is an amorphous, slightly colored powder. It is pulverized for use in the next step.

2. Preparation of 5'-Methoxylaudanosine

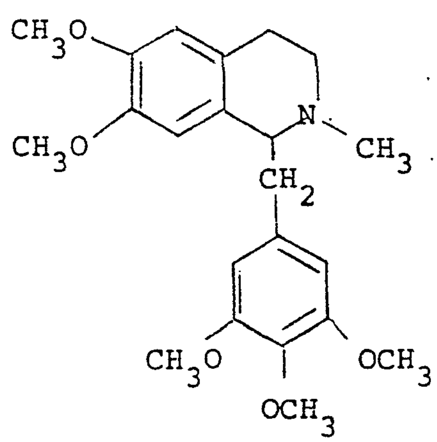

3,4-dimethoxyphenylethylamine and 3,4,5-trimethoxyphenylacetic acid are heated together at 165 - 190° in a flask until bubbling of water subsides. The product, 3,4,5-trimethoxybenzylacetylhomoveratrylamine, is recrystallized from methanol. Yield = 80 %. m.p. = 94°.

3,9 gm (10 mM) 3,4,5-trimethoxybenzylacetylhomoveratrylamine is refluxed in 15 ml toluene together with 5 ml $POCl_3$ for 2 hours. The settled semisolids are carefully separated ($POCl_3$ excess!) and the free base liberated by adding excess of NaOH and extracted with benzene. The product, 6,7-dimethoxy-1-(3',4',5'-trimethoxybenzyl) 3,4-dihydroisoquinoline is refluxed in acetone or benzene with an excess of methyl iodide. The quaternary salt, 6,7-dimethoxyl-(3',4',5'-trimethoxybenzyl)2-methyl 3,4-dihydroisoquinolinium iodide, precipitates out. m.p. = 224°.

1 gm (10 mM) 6,7-dimethoxy-1-(3',4',5'-trimethoxybenzyl) 2-methyl 3,4-dihydroisoquinolinium iodide is dissolved in 80 ml $H_2O$ and 16 ml concentrated HCl. Zinc dust (1,1 gm) is added in small portions to the boiling stirred solution. The yellow color disappears (reaction time 15 - 20 minutes). The mixture is filtered hot from some unreacted zinc and rendered alkaline with concentrated NaOH. It is impractical to filter the partly precipitated zinc hydroxide, so to avoid emulsions, the whole mixture is carefully shaken with chloroform. The residue of the chloroform solution is redissolved in ether and the ether insolubles are filtered off. The ether residue does not crystallize on standing. This amine is a gummy material which hardens on standing. The crude amine is used in the next step.

0010119

3. Preparation of N-(3-chloropropyl)5'-methoxylaudanosinium bromide

5'-Methoxylaudanosine 1,4 gm = 4 mM is dissolved in 8 ml dimethylformamide by warming slightly. 1-bromo-3-chloropropane 1,2 gm (about 100 % excess) is added and the mixture is left at room temperature for 5 days. (Sometimes part of the unreacted 5'-methoxylaudanosine crystallizes out, but eventually it redissolves).

The reddish-orange solution is treated with a large amount of ether and the precipitated gummy quaternary salt alt is decanted and slurried in fresh ether. After standing in ether for one day, low melting solids are obtained. Yield = 1,6 gm, about 80 % of theory.

4. Preparation of m-phenylene diacrylic diester of N-propyl 5'-methoxylaudanosine (LL39) (Horenstein - Pahlicke Ester Formation)

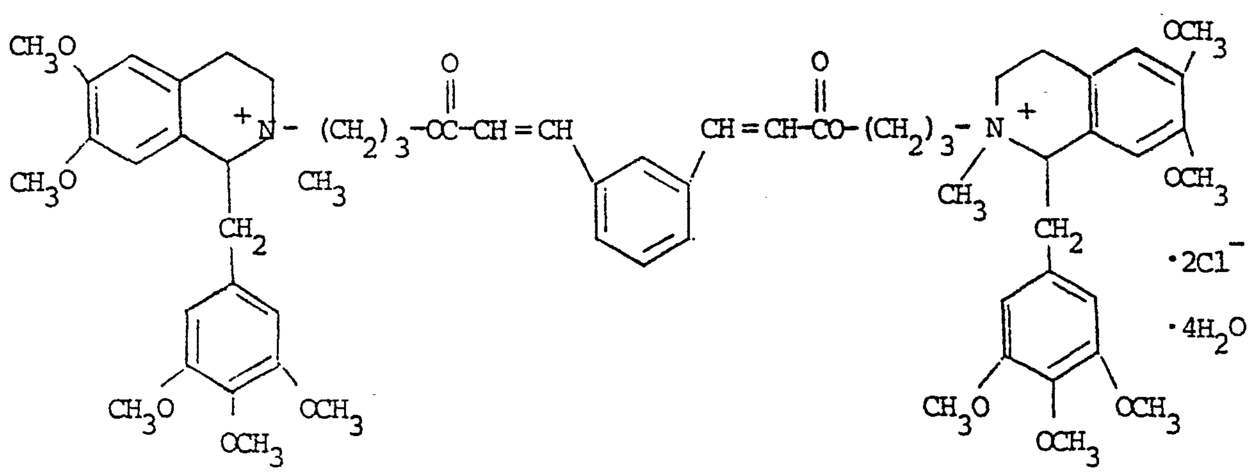

LL39

N-(3-chloropropyl)5'-methoxylaudanosinium bromide

| | | |
|---|---|---|
| N-(3-chloropropyl)5'-methoxylaudanosinium bromide | 2,1 gm = 4 mM |
| Silver m-phenylene diacrylate | 0,85 gm= 4 mM |
| $H_2O$ | about 150 ml |

The mixture is boiled in an open beaker for about 10 - 15 minutes, stirring by hand from time to time. At the boiling temperature the silver salt is slightly soluble and reacts with the quaternary bromide. The mixture is cooled to room temperature, filtered straight and the aqueous solution is evaporated to dryness in a large dish on a steam bath. Continued heating of the residue is done for about 2 hours on a steam bath (90°C), after which rearrangement to the ester is complete:

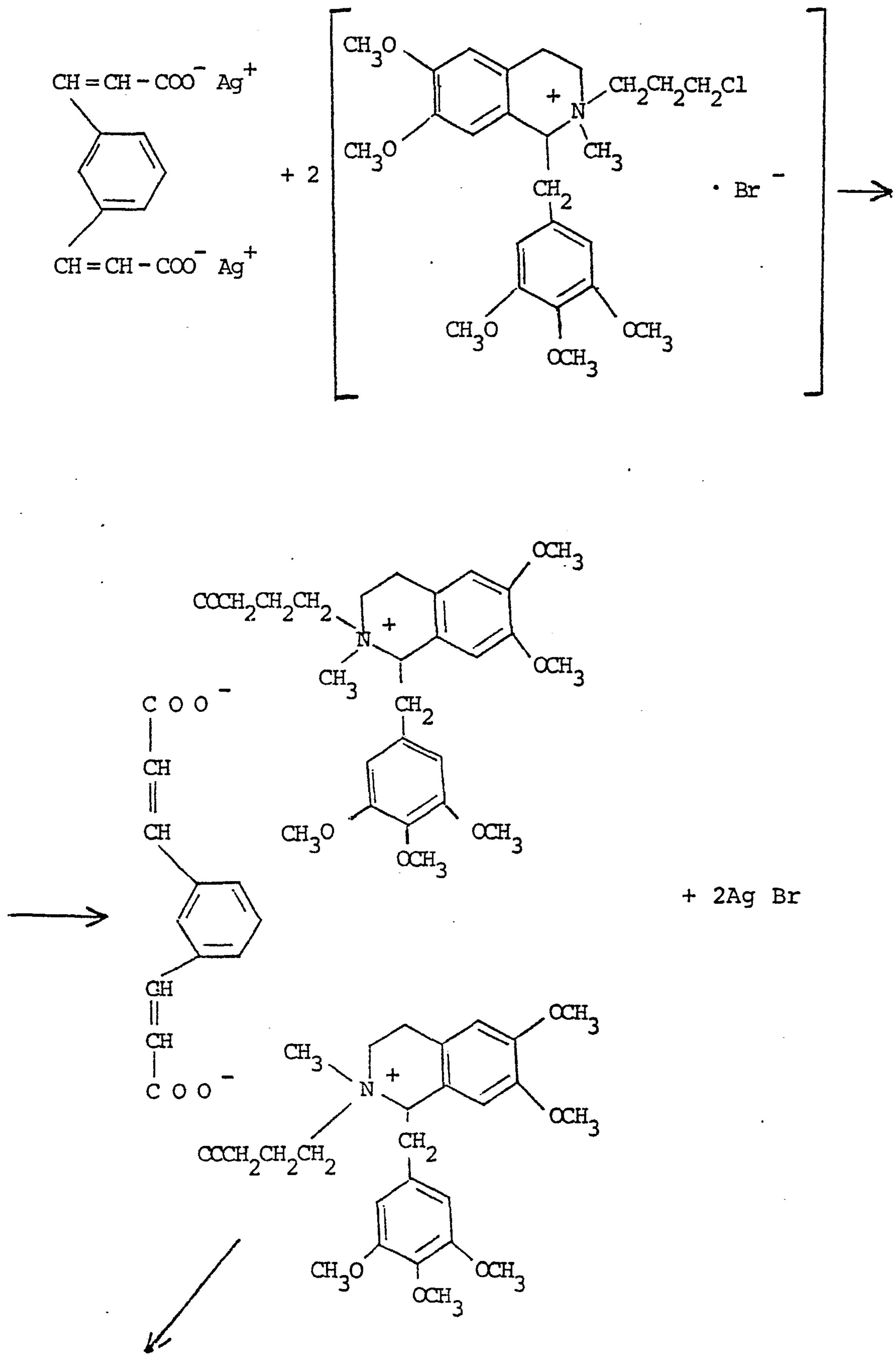


Rearranges to ester, LL-39

The amorphous residue is boiled with isopropanol (about 40 ml) and filtered hot from some trace mechanical impurities. Gums precipitate from the filtrate at room temperature and the precipitation is completed at about $-3^{o}$ overnight. The supernatant is decanted and the material is slurried in ethyl acetate twice. By now the gum is semisolid and can be filtered off. After careful drying at $75^{o}$ the gums become solids. At this stage they still probably retain water in varying degrees. Yield = 1,0 gm (about 40 %) Yields vary from batch to batch. M.P. = 80 - $90^{o}$ (decomposes).

A pharmaceutical formulation of LL39 is prepared as in Example 5 or 6.

EXAMPLE 11

The compound of Example 10 (LL-39) is converted to the dimesylate salt in an ion exchange reaction by reacting LL39 with silver mesylate. The dichloride (LL39) is dissolved in water as is the silver mesylate. The reaction mixture is stirred to form the silver chloride precipitate. The mixture is then filtered through filter paper to remove the silver chloride leaving the mesylate salt. Bis-3-[N-methyl-1-(3,4,5-trimethoxybenzyl) 6,7-dimethoxy-1,2,3,4-tetrahydroisoquinolinium] propyl m-phenylene-3,3'diacrylate dimesylate in solution. The water is then evaporated.

EXAMPLE 12

The compound of Example 10 (LL-39) is converted to the ditosylate salt in an ion exchange reaction by reacting LL39 with silver tosylate. The dichloride (LL39) is dissolved in water as is the silver tosylate. The reaction mixture is stirred to form the silver chloride precipitate. The mixture is then filtered through filter paper to remove the silver chloride leaving the tosylate salt. Bis-3-[N-methyl-1-(3,4,5-trimethoxybenzyl)6,7-dimethoxy-1,2,3,4-tetrahydroisoquinolinium] propyl m-phenylene-3,3'diacrylate ditosylate in solution. The water is tehn evaporated.

EXAMPLE 13

Following the procedures of the above examples the following compounds as dichlorides have been made.

| No. | MP (C) | M | B, C Relationship | W | Y | Z | $R_2$ | $R_2$ | $R_3$ | $R_4$ |
|---|---|---|---|---|---|---|---|---|---|---|
| GG32 | | 3 | para | CH=CH | $CH_3$ | H | H | $OCH_3$ | $OCH_3$ | H |
| GG45 | | 3 | para | " | " | $CH_3$ | " | " | " | " |
| GG46 | | 2 | para | " | " | " | " | " | " | " |
| GG122 | | 3 | meta | " | " | " | " | " | " | " |
| GG179 | | 2 | para | " | " | " | $OCH_3$ | " | " | " |
| HH79 | | 3 | para | " | " | " | " | " | " | " |
| MM168 | | 3 | para | " | " | " | H | " | " | $OCH_3$ |
| KK186 | | 3 | meta | " | " | 3,4-dimethoxy benzyl | H | " | " | H |
| LL39 | | 3 | meta | " | " | 3,4,5-tri-methoxy benzyl | H | " | " | H |
| NN106 | | 3 | meta | $CH_2$ | " | 3,4-dimethoxy benzyl | H | " | " | H |
| OO155 | | 3 | meta | $CH_2$ | " | 3,4,5-tri-methoxy benzyl | H | " | " | H |

Claims :

1. A compound of the formula (I)

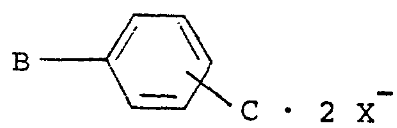

(I)

where B and C are para or meta and are each

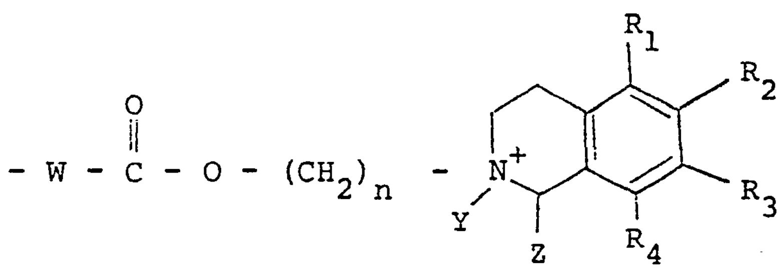

where W is CH = CH

$R_1$, $R_2$, $R_3$ and $R_4$ are the same or different and are each hydrogen or lower alkoxy of 1 to 4 carbon atoms,

Y is lower alkyl of 1 to 4 carbon atoms,

Z is hydrogen, lower alkyl of 1 to 4 carbon atoms, cyclopentyl, cyclohexyl, benzyl, or

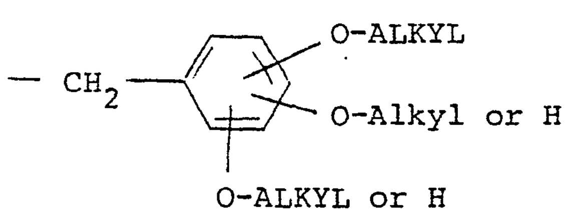

where ALKYL has 1 to 4 carbon atoms, n is 2, 3 or 4, provided that at least one of $R_1$ to $R_4$ is lower alkoxy and X is a pharmaceutically acceptable anion.

2. The compound of claim 1 in which X is iodide, mesylate, tosylate, bromide, chloride, sulfate, phosphate, hydrogen phosphate, acetate or propionate.

3. Bis-3-[N-methyl-1-(3,4,5-trimethoxybenzyl)6,7-dimethoxy-1,2,3,4-tetrahydroisoquinolinium]propyl m-phenylene-3,3'-diacrylate dimesylate.

4. Bis-3-[N-methyl-1-(3,4,5-trimethoxy-1,2,3,4-tetrahydroisoquinolinium]propyl p-phenylene-3,3'-diacrylate dimesylate.

5. Bis-3-[N-methyl-1-(3,4,5-trimethoxybenzyl) 6,6-dimethoxy-1,2,3,4-tetrahydroisoquinolinium] propyl m-phenylene-3,3'-diacrylate dichloride.

6. Bis-3-[N-methyl-1-(3,4,5-trimethoxy-1,2,3,4-tetrahydroisoquinolinium]propyl p-phenylene-3,3'-diacrylate dichloride.

7. Bis-3-[N-methyl-1-(3,,4,5-trimethoxybenzyl)6,7-dimethoxy-1,2,3,4-tetrahydroisoquinolinium]propyl m-phenylene-3,3-diacrylate ditosylate.

8. Bis-3-[N-methyl-1-(3,4,5-trimethoxy-1,2,3,4-tetrahydroisoquinolinium]propyl p-phenylene-3,3'-diacrylate ditosylate.

9. A sealed container containing the compound of anyone of claim 1 to 8.

10. A pharmaceutical preparation for parenteral administration comprising an effective neuromuscular blocking amount of the compound of anyone of claims 1 to 8 and pharmaceutically acceptable carrier therefore, preferably containing the blocking agent in an amount of 5 to 400 mg.

11. The compounds of claims 1 to 8 for use as a muscle relaxation agent.

12. A method of preparing a compound of formula (I)

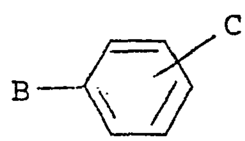 $2\ X^-$ (I)

where B and C are the same or different, B is para or meta to C, and each is

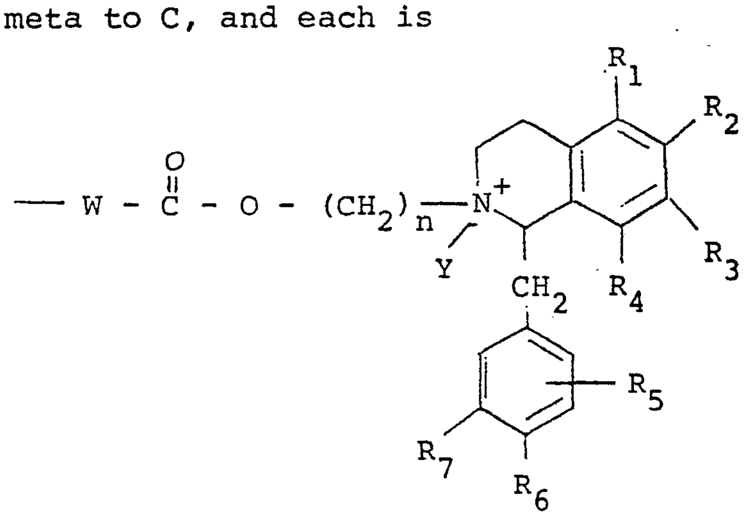

W is CH = CH

where

n is 2, 3 or 4;

$R_1$, $R_2$, $R_3$, $R_4$, $R_5$, $R_6$ and $R_7$ are the same or different and each is hydrogen or alkoxy of 1 to 4 carbon atoms;

Y is alkyl of 1 to 4 carbon atoms; and

$X^-$ represents one equivalent of pharmaceutically acceptable anion;

provided that at least one of $R_1$ to $R_4$ is alkoxy and at least one of $R_3$ to $R_7$ is alkoxy; characterised in that one:

a) reacts a species of formula

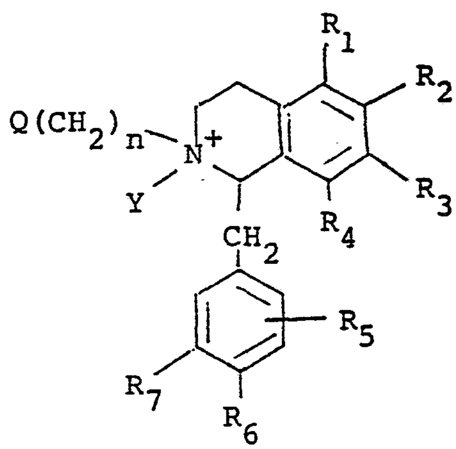

with a species of formula

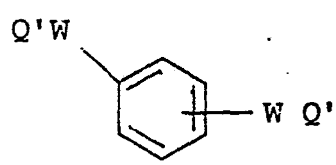

where n, W, Y and each of $R_1$ to $R_7$ have the same meaning as in formula (I) and Q and Q' are functional atoms or groups which react together to form an ester linkage;

or

b) quaternises a compound of formula

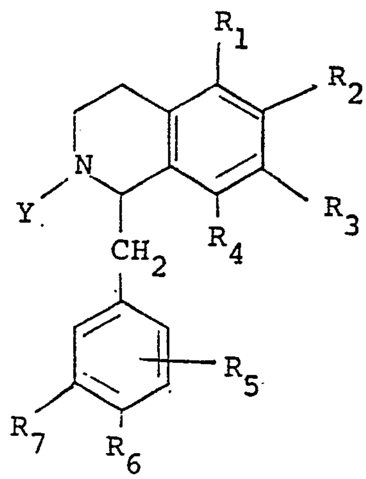

where Y and each of $R_1$ to $R_7$ have the same meaning as in formula (I), with a compound of formula

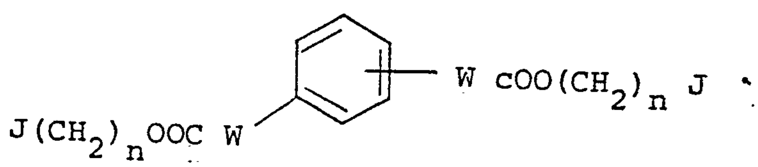

where J is halo and m and n habe the same meaning as in formula (I);

or

c) alkylates the corresponding ditertiary base of formula

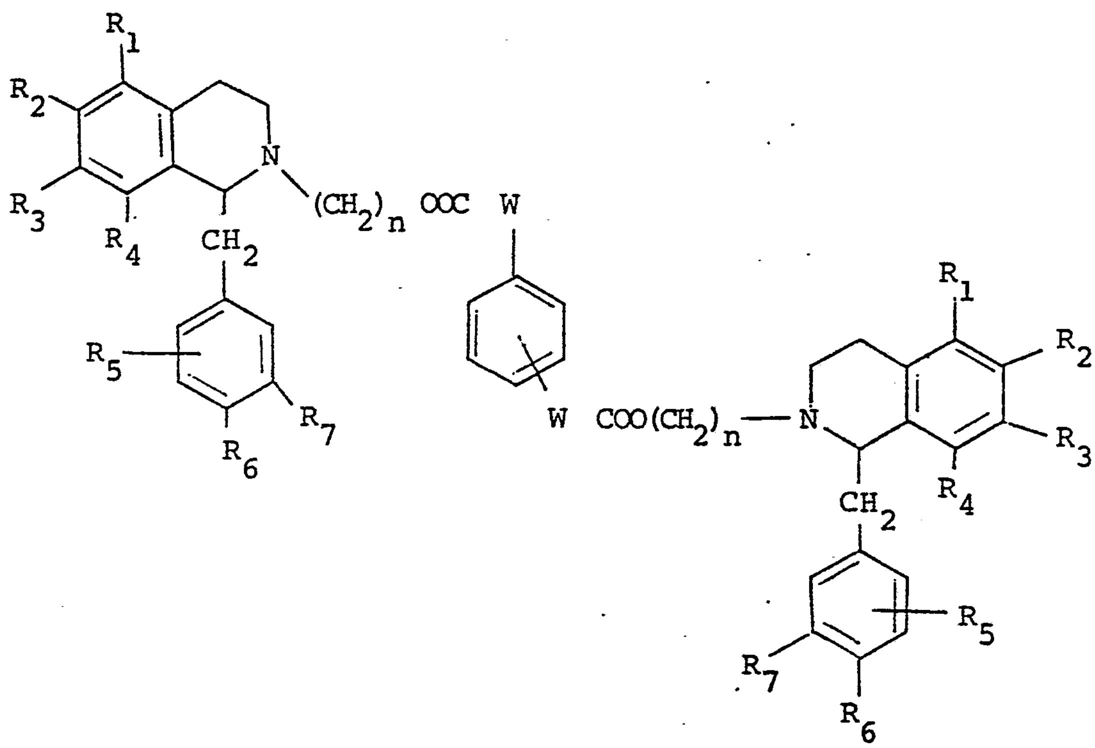

wherein n, W and each of $R_1$ to $R_7$ habe the same meaning as in formula (I), or the corresponding monotertiary base where a group Y as defined in formula (I) is attached to one of the isoquinolium nitrogen atoms, with an appropriate alkylating agent for introducing one or two Y groups as appropriate.

13. A method according to claim 12 (a) which comprises rearrangement of a salt of formula.

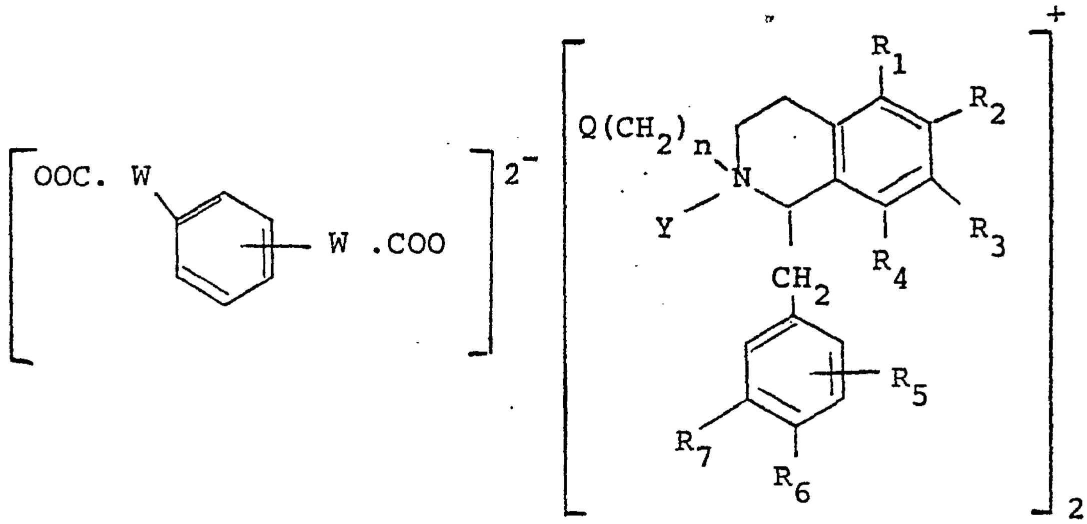

wherein each of W, n, Y and $R_1$ to $R_7$ have the same meaning as in formula (I) and Q is halo.

14. Compound of the formula (I)

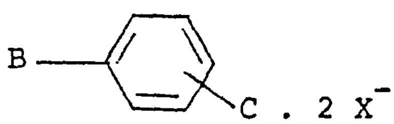

(I)

where B and C are para or meta and are each

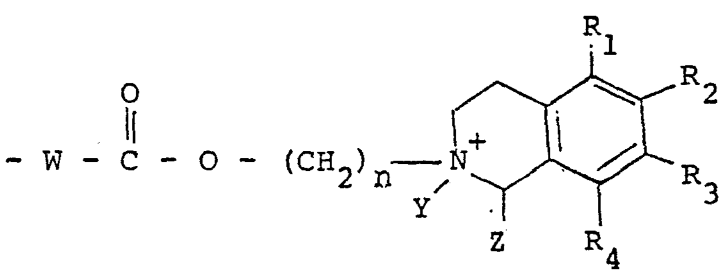

where W is $CH_2$
$R_1$, $R_2$, $R_3$ and $R_4$ are the same or different and are each hydrogen or lower alkoxy of 1 to 4 carbon atoms

Y is lower alkyl of 1 to 4 carbon atoms
Z is hydrogen, lower alkyl of 1 to 4 carbon atoms, cyclopentyl, cyclohexyl, benzyl, or

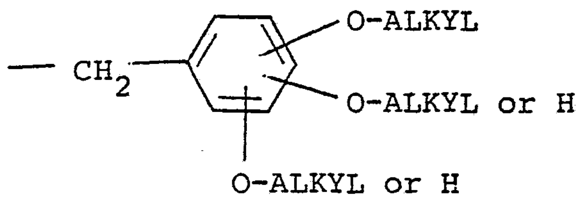

where ALKYL has 1 to 4 carbon atoms, n is 2, 3 or 4, provided that at least one of $R_1$ to $R_4$ is lower alkoxy and X is a pharmaceutically acceptable anion.

15. The compound of claim 14 in which Z is benzyl or benzyl substituted at 1, 2 or 3 positions with O-ALKYL where ALKYL contains 1 to 4 carbon atoms.

16. The compound of anyone of claims 14 to 15 in which X is iodide, mesylate, tosylate, bromide, chloride, sulfate, phosphate, hydrogen phosphate, acetate or propionate.

17. Bis-3-[N-methyl-1-(3,4-dimethoxybenzyl)-6,7-dimethoxy-1,2,3,4-tetrahydroisoquinolinium]propyl p-phenylene-3,3-diacryate .2X where X is chloride, mexylate or tosylate.

18. A sealed container containing the compound of anyone of claims 14 to 17.

19. A pharmaceutical preparation comprising an effective neuromuscular blocking amount of the compound of anyone of claims 14 to 17 and a pharmaceutically acceptable carrier therefore, preferably containing the blocking agent in an amount from 5 to 400 mg.

20. A method of preparing a compound of formula (I)

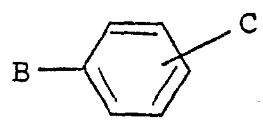 $2\ X^-$ (I)

where B and C are the same or different, B is para or meta to C, and each is

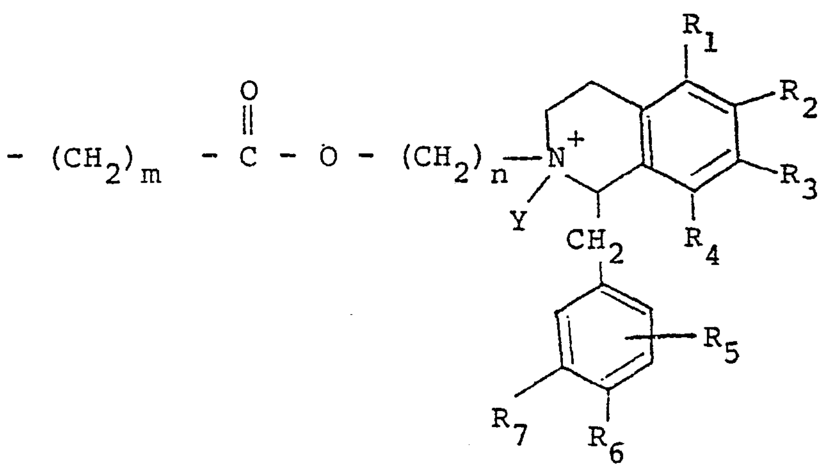

where m is 1
n is 2, 3 or 4;
$R_1$, $R_2$, $R_3$, $R_4$, $R_5$, $R_6$ and $R_7$ are the same or different and each is hydrogen or alkoxy of 1 to 4 carbon atoms;
Y is alkyl of 1 to 4 carbon atoms; and
$X^-$ represents one equivalent of pharmaceutically acceptable anion;

provided that at least one of $R_1$ to $R_4$ is alkoxy and at least one of $R_5$ to $R_7$ is alkoxy; characterized in that one:

a) reacts a species of formula

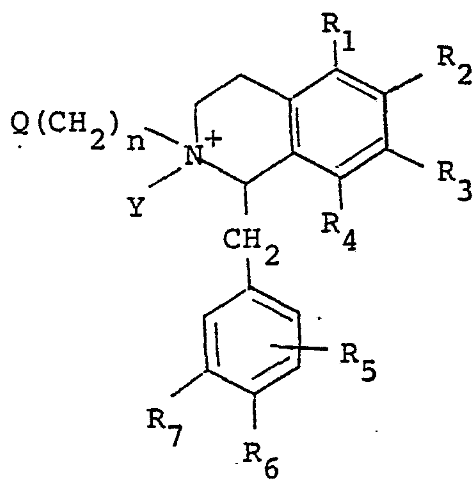

with a species of formula

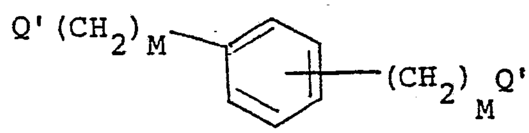

where n, M, Y and each of $R_1$ to $R_7$ have the same meaning as in formula (I) and Q and Q' are functional atoms or groups which react together to form an ester linkage;

or

b) quaternises a compound of formula

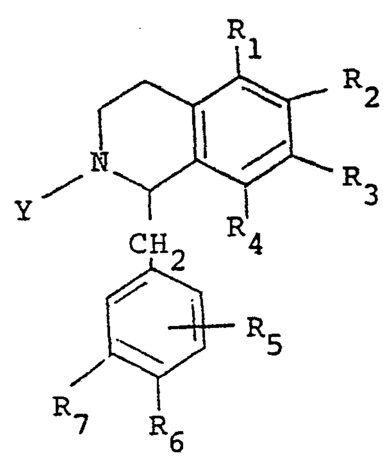

where Y and each of $R_1$ to $R_7$ have the same meaning as in formula (I), with a compound of formula

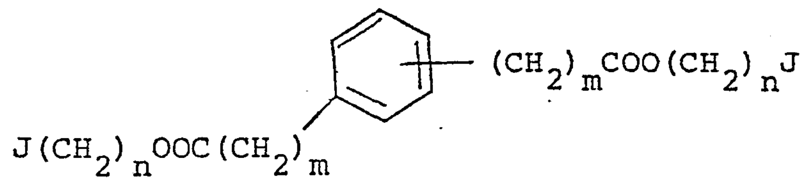

where J is halo and m and n have the same meaning as in formula (I);

or

c) alkylates the corresponding ditertiary base of formula

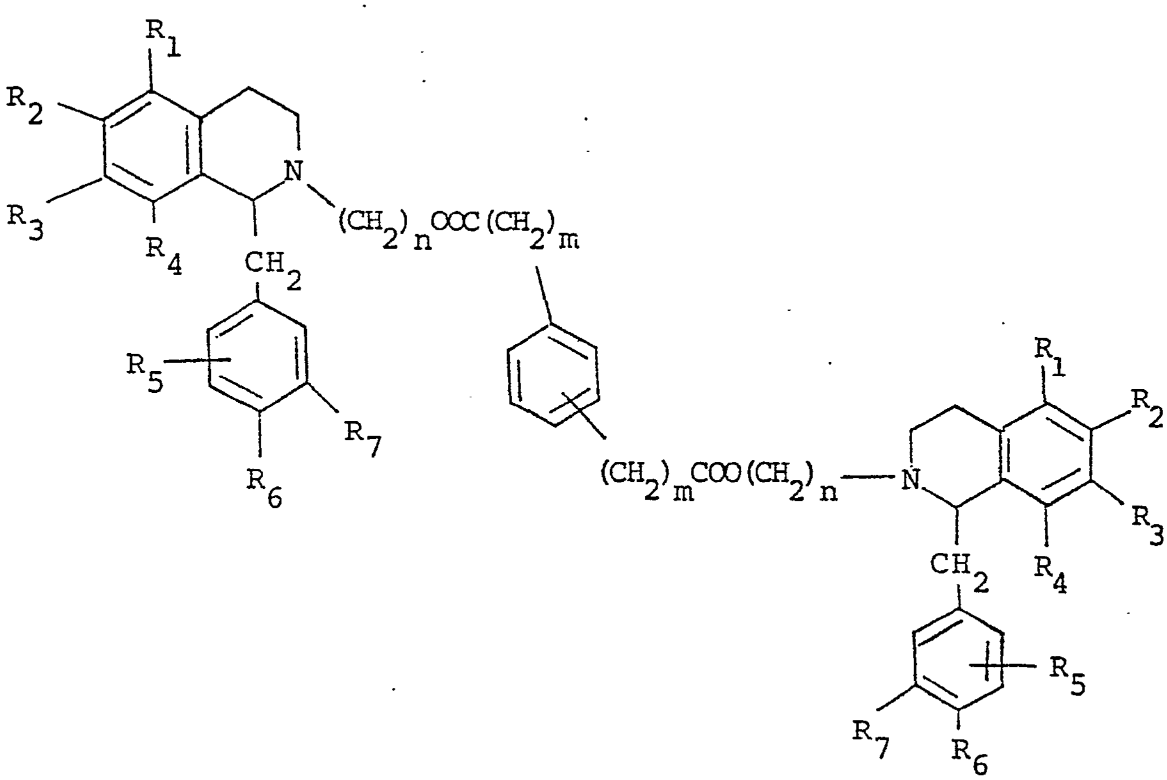

wherein n, m and each of $R_1$ to $R_7$ have the same meaning as in formula (I), or the corresponding monotertiary base where a group Y as defined in formula (I) is attached to one of the isoquinolium nitrogen atoms, with an appropriate alkylating agent for introducing one or two Y groups as appropriate.

21. A salt of formula

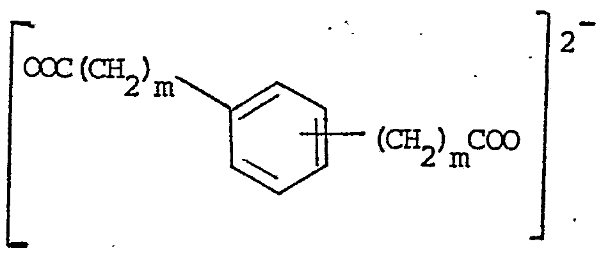

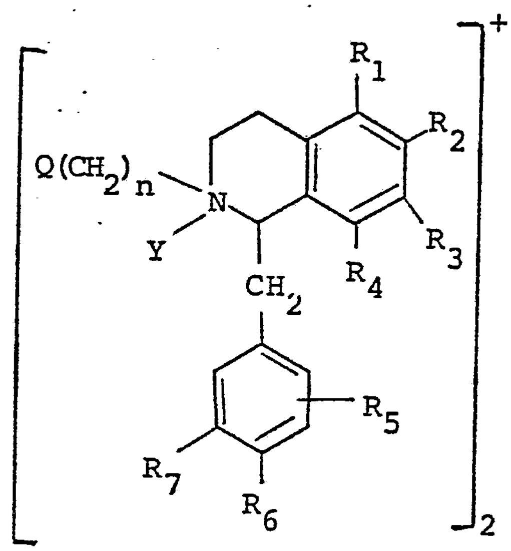

wherein each of n, m, Y and $R_1$ to $R_7$ has the same meaning as in formula (I) in claim 20 and Q is halo.

22. An acid of formula

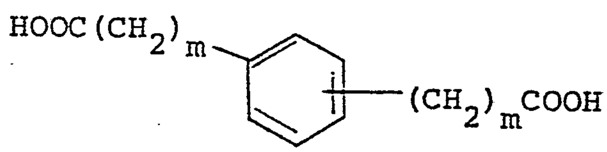

wherein m is 1, or an acid halide thereof.

23. A salt of formula

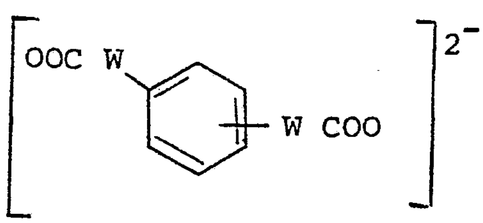

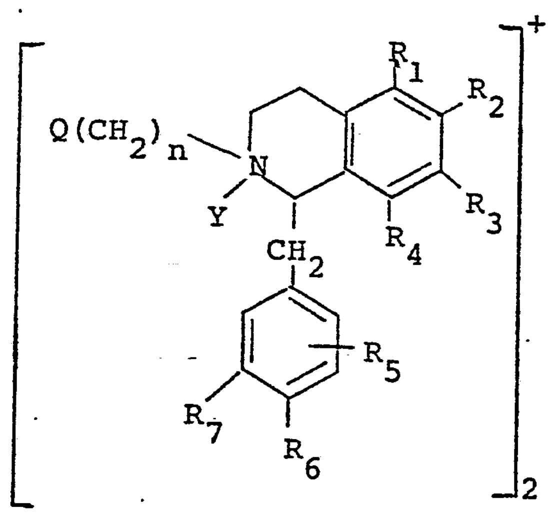

wherein each of n, W, Y and $R_1$ to $R_7$ has the same meaning as in formula (I) in claim 12 and Q is halo.

24. An acid of formula

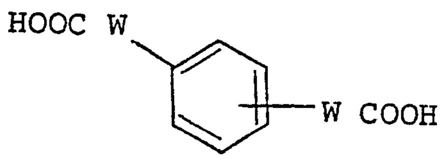

wherein W is CH = CH, or an acid halide thereof.

0010119

Application number

European Patent Office

# EUROPEAN SEARCH REPORT

EP 79 100 254.6

| DOCUMENTS CONSIDERED TO BE RELEVANT | | | CLASSIFICATION OF THE APPLICATION (Int. Cl.) |
|---|---|---|---|
| Category | Citation of document with indication, where appropriate, of relevant passages | Relevant to claim | |
| X | BEILSTEINS HANDBUCH DER ORGANISCHEN CHEMIE<br>4th edition, vol. 9,<br>1926, SPRINGER VERLAG, Berlin<br>* pages 874, 875, 914 *<br>-- | 22,24 | C 07 D 217/20<br>C 07 C 57/34<br>C 07 C 57/42<br>A 61 K 31/47 |
| X | BEILSTEINS HANDBUCH DER ORGANISCHEN CHEMIE<br>4th edition, 3rd supplement, vol. 9, part 5,<br>1971, SPRINGER VERLAG, Berlin, Heidelberg, New York<br>* pages 4435, 4436, 4438 *<br>-- | 24 | TECHNICAL FIELDS SEARCHED (Int.Cl.)<br>A 61 K 31/47<br>C 07 D 217/14<br>C 07 D 217/20 |
| X | Chemical Abstracts, Ninth Collective Index, vol. 76 to 85, 1972 to 1976<br>* page 3846 F *<br>-- | 22 | |
| | GB - A - 863 717 (ALLEN & HANBURYS)<br>* claim 4 *<br>-- | 12,20 | |
| E | GB - A - 2 002 758 (MASSACHUSETTS GENERAL HOSPITAL)<br>* complete document *<br>-- | 1-24 | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| A | DE - A1 - 2 655 883 (WELLCOME FOUNDATION<br>--<br>./.. | | |
| X | The present search report has been drawn up for all claims | | &: member of the same patent family, corresponding document |

| Place of search | Date of completion of the search | Examiner |
|---|---|---|
| Berlin | 26-11-1979 | FROELICH |

European Patent Office

# EUROPEAN SEARCH REPORT

0010119

Application number

EP 79 100 254.6

- page 2 -

| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| A | US - A - 2 662 083 (C.J. EASTLAND et al.)<br>-- | | |
| D,A | US - A - 3 491 099 (F.C. COPP)<br>---- | | |
| | | | TECHNICAL FIELDS SEARCHED (Int. Cl.3) |

EPO Form 1503.2 06.78